## Europäisches Patentamt

## European Patent Office

## Office européen des brevets

(19)

(11) Veröffentlichungsnummer: **0 034 739**
**A2**

(12) # EUROPÄISCHE PATENTANMELDUNG

(21) Anmeldenummer: **81100866.3**

(22) Anmeldetag: **07.02.81**

(51) Int. Cl.³: **B 01 D 19/02**

(30) Priorität: **12.02.80 DE 3005115**

(43) Veröffentlichungstag der Anmeldung:
**02.09.81 Patentblatt 81/35**

(84) Benannte Vertragsstaaten:
**AT CH FR GB LI SE**

(71) Anmelder: **Fried. Krupp Gesellschaft mit beschränkter Haftung**
**Altendorfer Strasse 103**
**D-4300 Essen 1(DE)**

(72) Erfinder: **Pilepp, Edwin**
**Roonstrasse 21**
**D-4250 Bottrop(DE)**

(72) Erfinder: **Scheffler, Ulrich**
**Laupendahler Landstrasse 19**
**D-4300 Essen(DE)**

(54) Verfahren zur Beeinflussung der Schaumbildung bei chemischen oder biochemischen Gas-Flüssigkeits-Reaktionen in Begasungsreaktoren und Begasungsreaktor zur Durchführung des Verfahrens.

(57) Verfahren zur Beeinflussung der Schaumbildung bei chemischen oder biochemischen Gas-Flüssigkeits-Reaktionen in Begasungsreaktoren und Begasungsreaktor zur Durchführung des Verfahrens.

In Begasungsreaktoren wird Schaum aus dem Bereich der Schaumzone abgesaugt und in den Flüssigkeits-bereich zurückgeführt. Dazu ist ein Ansaugtrichter vorgesehen, der im Bereich der oberhalb des Flüssigkeitsbereichs befindlichen Schaumzone liegt und an den sich ein Führungszylinder anschließt.

Zur Beeinflussung der Schaumbildung zumindest im wesentlichen ohne Einsatz chemischer Zusatzmittel und ohne wesentliche Erhöhung der Energiekosten wird der den Ansaugvorgang bewirkende Unterdruck durch Einleiten einer Strömung aus zumindest einem der für die Reaktion benötigten Fluide in einen Ansaugtrichter erzeugt; die Fluid-Strömung besteht aus dem zugeführten Reaktionsgas und/oder aus der zurückgeführten Reaktionsflüssigkeit. Vorteilhaft ist der Ansaugtrichter (10') und der Führungszylinder (10") als Mischdüse (10) mit einer Treibdüse (9) zu einer Ansaugeinheit (8) zusammengefaßt, die nach dem Ejektor-Prinzip arbeitet; die die Treibdüse (9) verlassende Fluid-Strömung erzeugt einen Unterdruck, unter dessen Wirkung Schaum aus der Schaumzone abgesaugt wird.

FIG.1

FRIED. KRUPP GESELLSCHAFT MIT BESCHRÄNKTER HAFTUNG
in Essen

Verfahren zur Beeinflussung der Schaumbildung bei
chemischen oder biochemischen Gas-Flüssigkeits-
Reaktionen in Begasungsreaktoren und Begasungsreaktor zur Durchführung des Verfahrens

Die Erfindung betrifft ein Verfahren zur Beeinflussung der Schaumbildung bei chemischen oder
biochemischen Gas-Flüssigkeits-Reaktionen in Begasungsreaktoren durch Absaugen des Schaums aus
den Bereich der Schaumzone und seine Rückführung
in den Flüssigkeitsbereich des Begasungsreaktors.
Gegenstand der Erfindung ist außerdem ein Begasungsreaktor mit einem Ansaugtrichter, der im Bereich
der oberhalb des Flüssigkeitsbereichs befindlichen
Schaumzone angeordnet ist und an den sich in Richtung auf den Flüssigkeitsbereich ein Führungszylinder anschließt.
Der Begasungsreaktor kann insbesondere als Umlaufreaktor ausgebildet sein, d.h. ein innerhalb des
Reaktorgefässes ortsfest angeordnetes Umlaufrohr
aufweisen, dem ein Gaseintragelement zugeordnet
ist. Die Erfindung kann jedoch auch bei Rührkesselreaktoren oder bei Blasensäulenreaktoren mit äußerem
oder ohne äußeren Zwangsumlauf zur Anwendung gelangen.

Bei den unter Gaszufuhr stattfindenden Reaktionen in Mehrphasengemischen (Gas/Flüssigkeit; Gas/Flüssigkeit/Feststoff) bemüht man sich im allgemeinen darum, den Stoffaustausch - dessen die Reaktionsgeschwindigkeit bestimmender Teilschritt in vielen Fällen der Übergang der gasförmigen Phase oder einer Teilkomponente dieser gasförmigen Phase in die Flüssigkeit ist - durch Erzeugung feindispergierter Gasblasen, durch hohe Turbulenz und durch möglichst lange Kontaktzeiten der miteinander in Reaktion zu bringenden Phasen zu verbessern. Die dadurch in vielen Fällen verursachte Schaumbildung kann in begrenztem Umfang wünschenswert sein, da sie die Phasengrenzfläche vergrößert und den Stoffaustausch zwischen den Phasen erleichtert. Andererseits führt die Schaumbildung jedoch zu einer Herabsetzung des Füllungsgrades des Reaktorgefässes und damit zu einer Verringerung der Ausbeute an Reaktionsprodukten pro Raum-Zeiteinheit. Insbesondere bei aeroben Fermentationen, wie beispielsweise bei der Produktion von Backhefe, müssen verfahrenstechnische Bedingungen geschaffen und eingehalten werden, die ein für die ablaufenden Reaktionen optimales Gleichgewicht zwischen Schaumbildung und Schaumzerfall zur Folge haben. Zur Beeinflussung und Stabilisierung der Schaumbildung werden u.a. chemische Mittel, insbesondere Gäröle eingesetzt. Diese Art und Weise der Schaumbekämpfung mit Hilfe von Fetten kann zu unkontrollierter Zugabe von Nährstoffen oder zu Schwierigkeiten bei der Aufarbeitung der Reaktionsprodukte führen.

Aber auch die nicht metabolisierbaren (d.h. nicht von der Zelle aufnehmbaren) Mittel zur Stabilisierung der Schaumbildung, die insbesondere bei biochemischen Verfahren zur Anwendung gelangen, führen in vielen Fällen zu einer ungünstigen Beeinflussung des Stoffübergangs, insbesondere des bei aeroben Reaktionen wichtigen Sauerstoffübergangs von der gasförmigen in die flüssige Phase. Dies hat zur Folge, daß bei sonst gleichen Verfahrensbedingungen die Konzentration des gelösten Sauerstoffs in der flüssigen Phase im Vergleich zu einer Verfahrensführung ohne chemische Zusatzmittel abnimmt und die Reaktion bzw. der Ablauf der mikrobiellen Stoffwechselvorgänge verlangsamt wird: Zur Erzielung gleicher Reaktionsgeschwindigkeiten und gleicher Ausbeuten pro Raum-Zeit-Einheit müssen also die Gaszufuhr und damit der Energieverbrauch erhöht werden. Abgesehen von den Kosten, die der Einsatz der chemischen Zusatzmittel und die eventuell mit der Aufbereitung der Reaktionsprodukte verbundenen Schwierigkeiten nach sich ziehen, entstehen unter Umständen zusätzliche Energiekosten in der Größenordnung von mehr als 30%.

Angesichts der soeben geschilderten Nachteile, die mit der Anwendung chemischer Zusatzmittel verbunden sind, versucht man, die Schaumbildung auf mechanischem Wege zu beeinflussen bzw. zu stabilisieren. Aus der DE-OS 25 39 797 sind ein Verfahren und eine Vorrichtung der eingangs genannten Gattung bekannt, bei denen Schaum mittels eines in den Flüssigkeitsbereich eintauchenden Umwälzbelüfters durch
den vorgeschalteten Ansaugtrichter mit sich anschließendem Führungszylinder angesaugt und unter
der Einwirkung des umlaufenden Rotors zerstört
wird. Der Nachteil dieses Standes der Technik besteht in der Verwendung eines besonders gestalteten
Umwälzbelüfters sowie - ebenso wie im Falle der
Schaumbekämpfung mittels einer Zentrifuge - darin,
daß im Begasungsreaktor befindliche Organismen
durch die auftretenden mechanischen Kräfte zerstört oder jedenfalls beschädigt werden. Schnell
umlaufende Umwälzbelüfter bzw. Zentrifugen sind
außerdem unter den Gesichtspunkten des Energieverbrauchs und der Schwingungsdämpfung verhältnismäßig aufwendig.

Zur Vermeidung der Nachteile, die mit dem Einsatz
schnell umlaufender Einrichtungen verbunden sind,
sind auch Vorschläge bekanntgeworden,die Schaumbildung in Begasungsreaktoren durch Rückführung aus
dem Flüssigkeitsbereich abgezogener Reaktionsflüssigkeit in den Bereich der Schaumzone zu beeinflussen. Die US-PS 4 058 481 beschreibt ein Verfahren, bei dem die eine Düse verlassende Reaktionsflüssigkeit mittels einer Prallplatte radial abgelenkt wird; die dabei entstehenden Flüssigkeitspartikel treffen mit hoher Geschwindigkeit auf den
Schaum auf und zerstören diesen.

Aus der DE-PS 943 161 sind ein Verfahren und eine
Vorrichtung zur Beeinflussung der Schaumbildung
bekannt, bei denen durch Absaugen und Rückführen von Reaktionsflüssigkeit innerhalb des Begasungsreaktors eine derartige Umwälzgeschwindigkeit eingestellt wird, daß die Geschwindigkeit des absinkenden Flüssigkeitsstromes größer ist als die Steiggeschwindigkeit schaumbildender Gasblasen in der Reaktionsflüssigkeit. Der Nachteil dieses bekannten Vorschlag ist darin zu sehen, daß eine bestimmte Sinkgeschwindigkeit aufrechterhalten werden muß, die nicht so groß sein darf, daß Gas mitgerissen wird.

Der Erfindung liegt die Aufgabe zugrunde, ein Verfahren und eine Vorrichtung der eingangs genannten Gattung zu entwickeln, die zumindest im wesentlichen ohne Einsatz chemischer Zusatzmittel zu geeigneten Verfahrensbedingungen führen und die ohne ins Gewicht fallende Erhöhung der Energiekosten wirksam sind.

Die gestellte Aufgabe wird durch ein Verfahren gelöst, welches im wesentlichen die Merkmale des Anspruchs 1 aufweist. Der der Erfindung zugrundeliegende Lösungsgedanke besteht danach darin, durch Einleiten einer geeigneten Fluid-Strömung in einen Ansaugtrichter den den Ansaugvorgang bewirkenden Unterdruck zu erzeugen; die Fluid-Strömung wird dabei unter Verwendung zumindest eines der für die Reaktion im Begasungsreaktor benötigten Fluide erzeugt, d.h. unter Verwendung eines Teilstroms des in den Begasungsreaktor eingeführten Reaktionsgases und/oder der aus dem Flüssigkeitsbereich abgezogenen Reaktionsflüssigkeit.

Um die Verhältnisse innerhalb des Begasungsreaktors an unterschiedliche Verfahrensbedingungen anpassen zu können, wird die Lage des Austritts der Fluid-Strömung in den Begasungsreaktor in Abhängigkeit von der Lage der Schaumzone verändert (Anspruch 2); auf die Weise ist es insbesondere möglich, die Ausdehnung der Schaumzone in der für den Reaktionsverlauf gewünschten Weise zu verändern.

Das Verfahren kann weiterhin so ausgestaltet sein, daß mittels der Fluid-Strömung gleichzeitig der Wärmehaushalt des Begasungsreaktors gesteuert wird (Anspruch 3).

Um die mit der Erzeugung der Fluid-Strömung verbundenen Mehrkosten in engen Grenzen zu halten, kann das neu vorgeschlagene Verfahren zusätzlich dadurch gekennzeichnet sein, daß in Ausnahmefällen zusätzlich zur Absaugung mittels der Fluid-Strömung chemische Zusatzmittel zur Beeinflussung der Schaumbildung in den Begasungsreaktor eingegeben werden. Die Absaugung mittels der Fluid-Strömung ist dabei an den normalerweise zu erwartenden Reaktionsverlauf angepaßt; falls sich aus irgendwelchen Gründen übermäßig viel Schaum bildet, kann dieser mittels der an sich bekannten chemischen Zusatzmittel bekämpft werden.

Der zur Lösung der gestellten Aufgabe geeignete Begasungsreaktor weist im wesentlichen die Merkmale des Anspruchs 4 auf. Wesentlicher Bestandteil des Begasungsreaktors ist danach eine nach dem Ejektor-Prinzip arbeitende Ansaugeinheit, die aus einer mit einem Fluid beaufschlagten Treibdüse und einer Mischdüse zusammengesetzt ist; die Mischdüse besteht ihrerseits aus einem Ansaugtrichter und einem sich anschließenden Führungszylinder. Das aus der Treibdüse austretende Fluid (Reaktionsgas und/oder Reaktionsflüssigkeit) erzeugt im Bereich der Mischdüse einen Unterdruck, über welchen Schaum aus der Schaumzone abgesaugt und durch den Führungszylinder dem Flüssigkeitsbereich wieder zugeführt wird.

Der Begasungsreaktor kann insbesondere dadurch ausgestaltet sein, daß er die Mekmale zumindest eines der Ansprüche 5 bis 12 aufweist.

Die getrennte Beweglichkeit der Mischdüse und der Treibdüse (Anspruch 10) ermöglicht es, einerseits den Saugvorgang zu beeinflussen und andererseits die Lage der Ansaugeinheit im Bereich der Schaumzone und/oder bezüglich der Flüssigkeit innerhalb des Reaktorgefässes zu verändern.

Die Ansaugeinheit kann je nach den gegebenen Verfahrensbedingungen an einen Flüssigkeitskreislauf (Anspruch 6) oder an einen Gaskreislauf (Anspruch 7) angeschlossen sein; es ist jedoch - beispielsweise zur Verbesserung der Begasung der Flüssigkeit - möglich, die Merkmale der Ansprüche 6 und 7 zu kombinieren, d.h. die Ansaugeinheit - zweckmäßigerweise unter Zwischenschaltung geeigneter Regelventile - gleichzeitig an einen Flüssigkeits- und Gaskreislauf anzuschließen:

Bei einer derartigen Ausführungsform des Begasungsreaktors wird die Fluid-Strömung also aus einem Gas-Flüssigkeits-Gemisch mit veränderbarer Zusammensetzung gebildet.

Die Mischdüse ist vorteilhaft zumindest teilweise als Diffusor ausgebildet, erweitert sich also auf dem in Frage kommenden Abschnitt in Strömungsrichtung (Anspruch 11).

Der Austritt der Treibdüse nimmt vorzugsweise eine Lage ein, in welcher er in Höhe des Übergangsbereichs zwischen dem Ansaugtrichter und dem Führungszylinder gehalten ist (Anspruch 12); abhängig von den sonstigen Verfahrensbedingungen kann der Austritt der Treibdüse jedoch auch geringfügig oberhalb oder geringfügig unterhalb des erwähnten Übergangsbereichs liegen.

Die Erfindung wird nachfolgend anhand zweier in der Zeichnung dargestellter Ausführungsbeispiele im e.nzelnen erläutert.

Es zeigen:

Fig. 1 schematisch einen Vertikalschnitt durch einen lotrecht stehenden Umlaufreaktor, dem aus dem Flüssigkeitsbereich abgezogene Reaktorflüssigkeit von außen zur Erzeugung eines Unterdrucks zugeführt wird,

Fig. 2 schematisch einen Vertikalschnitt durch einen lotrecht stehenden Umlaufreaktor mit zusätzlicher Gaszuführung und

Fig. 3 schematisch in vergrößertem Maßstab die Ausbildung einer Ansaugeinheit.

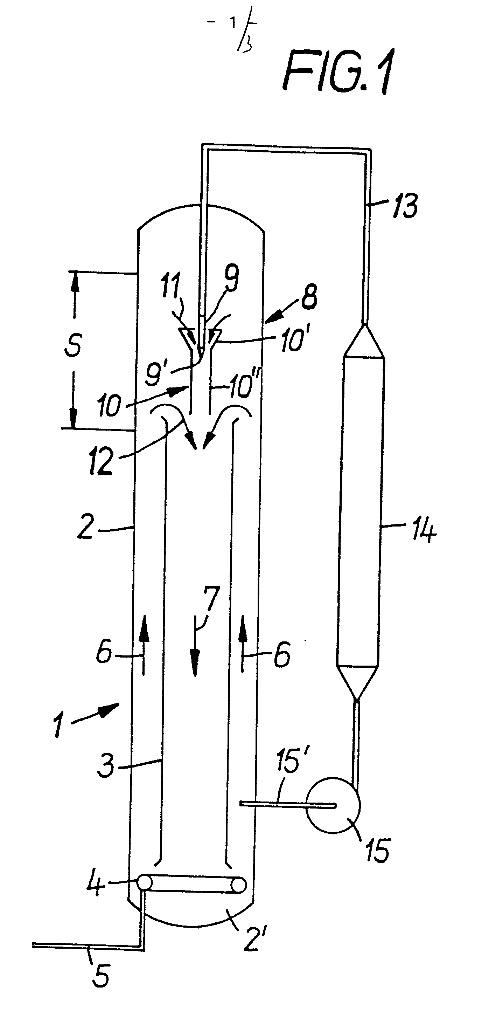

Der Begasungsreaktor 1 weist als Hauptbestandteile ein zylinderförmiges Reaktorgefäß 2 und einen konzentrisch in dessen Innenraum befestigtes Umlaufrohr 3 auf, das sich im Bereich seiner Endabschnitte kegelförmig erweitert. Unterhalb des Umlaufrohres 3 befindet sich im Bereich des unteren Endabschnitts 2' des Reaktorgefäßes ein Gaseintragelement 4 in Form eines mit nach oben gerichteten (nicht dargestellten) Bohrungen versehenen Ringrohres, welches über eine Leitung 5 an ein (nicht dargestelltes) Gas-Drucknetz angeschlossen ist (Fig. 1).

Durch Einleiten des Gases in das Reaktorgefäß 2 wird ein Lösungsumlauf hervorgerufen, der außerhalb des Umlaufrohres 3 durch nach oben gerichtete Pfeile 6 und innerhalb des Umlaufrohres durch einen nach unten gerichteten Pfeil 7 angedeutet ist.

Die Schaumzone des Begasungsreaktors 1 liegt im wesentlichen oberhalb des oberen Endes des Umlaufrohres 3; sie ist mit S gekennzeichnet. Im Bereich der Schaumzone S ist innerhalb des Reaktorgefäßes eine Ansaugeinheit 8 angeordnet, die aus einer Treibdüse 9 und einer Mischdüse 10 besteht; letztere setzt sich aus einem Ansaugtrichter 10' und aus einem sich an diesen anschließenden Führungszylinder 10" zusammen.

Die Mischdüse 10 ist bezüglich der Treibdüse 9 so angeordnet, daß der Ansaugtrichter 10' in der Nähe des Austritts 9' der Treibdüse 9 endet. Die Länge des Führungszylinders 10" ist zweckmäßigerweise so gewählt, daß der über den Ansaugtrichter 10' angesaugte Schaum (angedeutet durch Pfeile 11) mit Sicherheit in den Flüssigkeitsbereich innerhalb des Umlaufrohres 3 zurückgeführt wird. Die Bewegung der Flüssigkeit im Bereich des oberen Endes des Umlaufrohres 3' ist durch Pfeile 12 angedeutet.

Mit Hilfe der Treibdüse 9 - die vorzugsweise eine Strahlaustrittsgeschwindigkeit im Bereich zwischen 10 bis 40 m/s (Meter pro Sekunde) erzeugt - wird im Bereich der Mischdüse 10 der zur Ansaugung und Abführung des Schaumes erforderliche Unterdruck erzeugt. Überraschenderweise kann bei den üblicherweise vorkommenden Schaumarten die Absaugung in der Weise bewirkt werden, daß in der Schaumzone S keine Hohlräume entstehen: bei geeigneter Ausbildung der Treibdüse 9, des Ansaugtrichters 10' und des Führungszylinders 10" zeigt der Schaum ein einer Flüssigkeit ähnliches Strömungsverhalten.

Die Treibdüse 9 ist über eine Förderleitung 13, einen Wärmetauscher 14 und eine Pumpe 15 mit zugehöriger Ansaugleitung 15' mit dem Flüssigkeitsbereich des Reaktorgefäßes 2 verbunden; die Ansaugleitung 15' mündet also in der Nähe des Ringrohres 4 in das Reaktorgefäß ein.
Die von der Pumpe 15 zur Erzeugung der Flüssigkeitsströmung verbrauchte Energie macht nur einen geringen Bruchteil der Energie aus, die durch Verringerung der zur Begasung des Begasungsreaktors 1 erforderlichen Gasmenge eingespart werden kann. Die dargestellte Ausführungsform ist auch deshalb besonders wirtschaftlich, weil Begasungsreaktoren in vielen Fällen ohnehin einen äußeren Flüssigkeitskreislauf (bzw. Lösungskreislauf) aufweisen, der dazu dient, über den Wärmetauscher 14 Reaktionswärme zu- oder abzuführen oder im Reaktorgefäß 2 eine für den Reaktionsablauf günstige Temperatur einzustellen. Bei derartigen, bereits einen äußeren Flüssigkeits- oder Lösungskreislauf aufweisenden Begasungsreaktoren muß zum Betrieb der zusätzlich vorgesehenen Ansaugeinheit 8 die ohnehin vorhandene Pumpe 15 lediglich für einen etwas höheren Gegendruck ausgelegt sein, um den durch die Treibdüse 9 verursachten Druckverlust auszugleichen.

Im Gegensatz zu der in Fig. 1 dargestellten Ausführungsform kann die Treibdüse 9 auch an einen Gaskreislauf angeschlossen sein, insbesondere durch eine (nicht dargestellte) Zusatzleitung, die in die Leitung 5 einmündet; in diesem Falle wird die die Absaugung bewirkende Fluid-Strömung also mittels eines Gases erzeugt, welches sowieso für den Betrieb des Begasungsreaktors zur Verfügung steht.

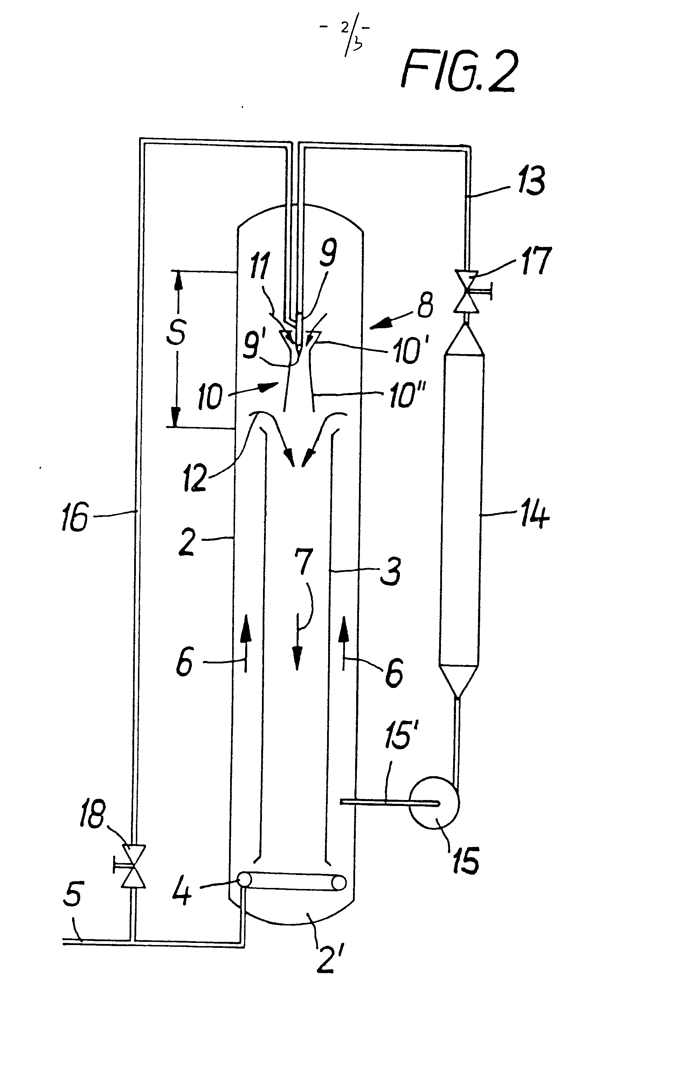

Bei der in Fig. 2 dargestellten Ausführungsform des Erfindungsgegenstandes ist die Treibdüse 9 nicht nur an die Förderleitung 13 angeschlossen, sondern über eine Zusatzleitung 16 mit der Leitung 5 verbunden. Die Leitung 13 und die Zusatzleitung 16 sind mit einem Regelventil 17 bzw. 18 ausgestattet, mit denen die in die Treibdüsen 9 einströmende Mischung aus Gas und Flüssigkeit in dem gewünschten Umfang verändert werden kann. Die Zusatzleitung 16 ist im Bereich der Treibdüse 9 zweckmäßigerweise so ausgebildet und angeordnet, daß sie konzentrisch von der Förderleitung 13 umschlossen ist.

Bei der soeben beschriebenen Ausführungsform, kann - abhängig von der Einstellung der Ventile 17 und 18 - die Fluid-Strömung im Grenzfall entweder nur mittels Gas oder nur mittels Flüssigkeit erzeugt werden.
Der Ansaugtrichter 10' geht mit geringem Abstand oberhalb des Austritt 9' der Treibdüse 9 in den Führungszylinder 10" über, dessen unterer Abschnitt als sich kegelförmig erweiternder Diffusor ausgebildet ist.

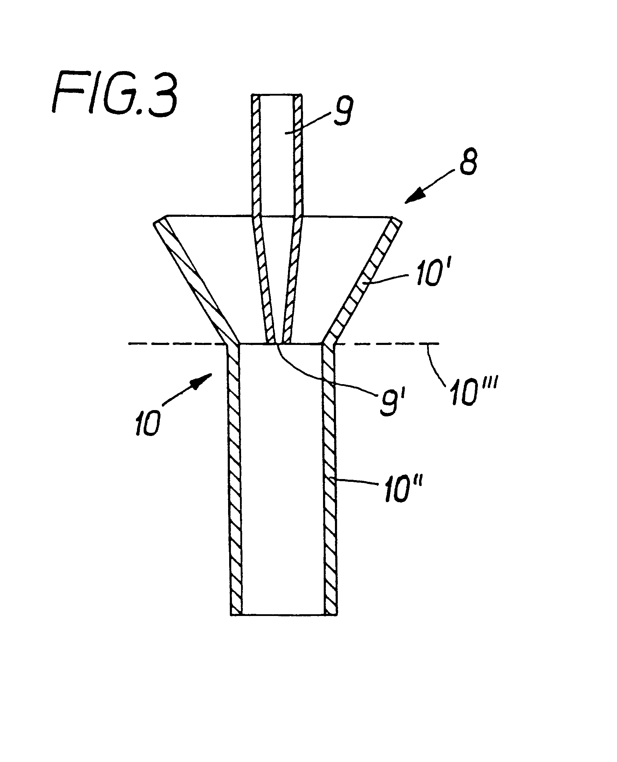

Die in Fig. 3 dargestellte bevorzugte Ausführungsform der Ansaugeinheit 8 wurde zur Schaumbekämpfung in einem der kontinuierlichen Hefeanzucht dienenden Reaktorgefäß mit einem Fassungsvermögen von 2 m$^3$ eingesetzt. Der Innendurchmesser der Treibdüse 9 beträgt 25 mm, derjenige des Austritts 9' etwa 5 bis 10 mm; die kegelförmige Wandung der Treibdüse 9 schließt mit der Lotrechten einen Winkel von 8$^{\circ}$ ein. Die Wandung des Ansaugtrichters 10' ist gegenüber der Lotrechten um 60$^{\circ}$ geneigt; die Länge des sich anschließenden Führungszylinders 10" mit einem Innendurchmesser zwischen etwa 40 bis 50 mm liegt bei etwa 800 bis 1000 mm.
Eine besonders gute Saugwirkung der Ansaugeinheit 8 wurde in den Fällen festgestellt, in denen der Austritt 9' der Treibdüse 9 nicht mehr als 10 mm oberhalb oder unterhalb des Übergangsbereichs zwischen dem Ansaugtrichter 10' und dem Führungszylinder 10" lag; die Lage des Übergangsbereichs ist durch eine Linie 10''' angedeutet. Die der Treibdüse 9 über die Förderleitung 13 (Vgl. Fig. 1) zugeführte Lösungsmenge betrug etwa 1,9 $m^3$/h bei einem Druck von etwa 2,1 bar; die Austrittsgeschwindigkeit aus der Treibdüse 9 wurde mit etwa 19,4 m/s bestimmt.
Während die Treibdüse 9 in dem (nicht dargestellten) Reaktorgefäß unbeweglich gehalten war, konnte die Mischdüse 10 mit den Bestandteilen 10' und 10" mittels einer Klemmvorrichtung in ihrer Höhenlage verändert werden.

Der Erfindungsgegenstand ist nicht auf die Anwendung bei Umlaufreaktoren beschränkt; er kann vielmehr auch bei Begasungsreaktoren anderer Bauart zum Einsatz gelangen, insbesondere bei Rührkesselreaktoren oder bei Blasensäulenreaktoren.

Die Vorteile des Erfindungsgegenstandes werden nachfolgend anhand eines Anwendungsbeispiels erläutert: Bei Fermentationsversuchen (Anzucht von Hefe auf Melasse als Nährlösung) wurde neben einer konventionellen Einrichtung zur Schaumbeeinflussung des mittels chemischer Mittel in der Schaumzone eines Umlaufreaktors eine abschaltbare Ansaugeinheit der bereits beschriebenen Ausführung eingebaut (vgl. dazu Fig. 1 und Fig. 3). Zum Nachweis der Wirksamkeit der Schaumbeeinflussung mittels der Ansaugeinheit wurde der Umlaufreaktor jeweils mehrere Stunden mit in Betrieb befindlicher bzw. bei abgeschalteter Ansaugeinheit gefahren; die in beiden Fällen zur Begrenzung der Schaumbildung erforderlichen Mengen an chemischen Zusatzmitteln wurden aufgezeichnet.

Obwohl bei den Versuchen zur Erzeugung der die Ansaugung bewirkenden Flüssigkeitsströmung lediglich eine Pumpe mit niedriger Förderleistung zum Einsatz kam, die von dem bereits vorhandenen Kühlkreislauf übernommen wurde, konnte bei eingeschalteter Ansaugeinheit der Verbrauch an chemischen Zusatzmitteln um 40 bis 70 % gesenkt werden; gleichzeitig wurde angesichts der verbesserten Übertragungsbedingungen für Sauerstoff eine höhere Konzentration an gelöstem Sauerstoff in der flüssigen Phase ermittelt.

In Abhängigkeit von den sonstigen Randbedingungen, unter denen die Versuche stattfanden, konnte die Belüftungsintensität (d. h. die dem Begasungsreaktor pro Raum- und Zeit-Einheit zugeführte Menge an komprimierter Luft) um 10 % bis nahezu 50 % gesenkt werden. Es ließen sich also erhebliche Einsparungen an Energie erzielen, ohne die für den Prozeßablauf wesentliche Konzentration an gelöstem Sauerstoff in der Fermentationslösung gegenüber einer Betriebsweise des Umlaufreaktors mit höherer Belüftungsintensität, aber bei abgeschalteter Ansaugeinheit, abzusenken.

Die mit der Erfindung gegebene Lehre kann also auch dazu benutzt werden, den Lösungsdruck des an der Reaktion beteiligten Gases oder einer Gaskomponente zu erhöhen und dadurch die stattfindende Reaktion zu beschleunigen. Das Erfindungsprinzip besteht in der Arbeitsweise einer Ansaugeinheit, die - ähnlich einer Zweistoffdüse - mit einem von außen zugeführten Treibmittel ein anderes Mittel, nämlich Schaum, ansaugt und weiterleitet.

Patentansprüche:

1. Verfahren zur Beeinflussung der Schaumbildung bei chemischen oder biochemischen Gas-Flüssigkeits-Reaktionen in Begasungsreaktoren durch Absaugen des Schaumes aus dem Bereich der Schaumzone und seine Rückführung in den Flüssigkeitsbereich des Begasungsreaktors, dadurch gekennzeichnet, daß der den Ansaugvorgang bewirkende Unterdruck durch Einleiten einer Strömung aus zumindest einem der für die Reaktion benötigten Fluide - einem Teilstrom des zugeführten Reaktionsgases und der aus dem Flüssigkeitsbereich abgezogenen Reaktionsflüssigkeit - in einen Ansaugtrichter erzeugt wird.

2. Verfahren nach Anspruch 1, dadurch gekennzeichnet, daß die Lage des Austritts der Fluid-Strömung in den Begasungsreaktor in Abhängigkeit von der Lage der schaumzone verändert wird.

3. Verfahren nach einem der Ansprüche 1 bis 2, dadurch gekennzeichnet, daß mittels der Fluid-Strömung gleichzeitig der Wärmehaushalt des Begasungsreaktors gesteuert wird.

4. Begasungsreaktor zur Durchführung des Verfahrens nach einem der Ansprüche 1 bis 3, mit einem Ansaugtrichter, der im Bereich der oberhalb des Flüssigkeitsbereichs befindlichen Schaumzone angeordnet ist und an den sich in Richtung auf den Flüssigkeitsbereich ein Führungszylinder anschließt, dadurch gekennzeichnet, daß der Ansaugtrichter (10') und der Führungszylinder (10") als Mischdüse (10) mit einer Treibdüse (9) zu einer Ansaugeinheit (8) zusammengefaßt ist, die nach dem Ejektor-Prinzip arbeitet.

5. Begasungsreaktor nach Anspruch 4, dadurch gekennzeichnet, daß die Ansaugeinheit (8) innerhalb des Reaktorgefäßes (2) höhenverstellbar ist.

6. Begasungsreaktor nach einem der Ansprüche 4 bis 5, dadurch gekennzeichnet, daß die Ansaugeinheit (8) über eine Förderleitung (13) mit dem mit Flüssigkeit gefüllten Bereich des Reaktorgefäßes (2) in Verbindung steht.

7. Begasungsreaktor nach einem der Ansprüche 4 bis 6, dadurch gekennzeichnet,daß die Ansaugeinheit (8) an das Drucknetz (Leitung 5) für ein Gaseintragelement (4) angeschlossen ist, über welches dem Flüssigkeitsbereich Reaktionsgas zuführbar ist.

8. Begasungsreaktor nach einem der Ansprüche 4 bis 7, dadurch gekennzeichnet, daß die Förderleitung (13) Bestandteil eines Wärmetauschers (14) ist.

9. Begasungsreaktor nach einem der Ansprüche 4 bis 8, mit einem im Reaktorgefäß angeordneten Umlaufrohr und einem im Bereich des unteren Endabschnitts des Reaktorgefäßes befindlichen Gaseintragelement, dadurch gekennzeichnet, daß zumindest die Ansaugöffnung der Ansaugeinheit (8) oberhalb des Umlaufrohres (3) liegt.

10. Begasungsreaktor nach einem der Ansprüche 4 bis 9, dadurch gekennzeichnet, daß die Treibdüse (9) und die Mischdüse (10) unabhängig voneinander innerhalb des Reaktorgefäßes (2) höhenverstellbar sind.

11. Begasungsreaktor nach einem der Ansprüche 4 bis 10, dadurch gekennzeichnet, daß die Mischdüse (10) zumindest teilweise als Diffusor ausgebildet ist (Fig. 2).

12. Begasungsreaktor nach einem der Ansprüche 4 bis 11, dadurch gekennzeichnet, daß der Austritt (9') der Treibdüse (9) in Höhe des Übergangsbereichs (10''') zwischen dem Ansaugtrichter (10') und dem Führungszylinder (10'') gehalten ist.

- 1/3

# FIG.1

- 2/3 -

*FIG.2*

FIG.3